# EUROPEAN PATENT APPLICATION

(11) **EP 4 246 531 A1**
(43) Date of publication of application: **20.09.2023**
(21) Application number: 21891432.3
(22) Date of filing: 09.08.2021
(51) Int. Cl.: G16H 50/80

(54) **INFORMATION PROCESSING SYSTEM, INFORMATION PROCESSING METHOD, AND COMPUTER PROGRAM**

(30) Priority: 13.11.2020 JP 2020189087
(71) Applicant: IoT-EX Inc., Chiyoda-ku Tokyo 101-0043 (JP)
(72) Inventor: MATSUMURA, Jun, Tokyo 101-0043 (JP)
(74) Representative: REHBERG HÜPPE + PARTNER
(86) International application number: PCT/JP2021/029465
(87) International publication number: WO 2022/102184

(57) **Abstract**

Specifying of a behavior history of an infected person and extracting of a person having a high likelihood of having been infected with an infectious disease from the infected person are performed easily and reliably. An information processing apparatus according to the present disclosure is an information processing system including: a second information processing apparatus having a function of receiving a signal transmitted from a first information processing apparatus; and a server apparatus that is connectable to at least the second information processing apparatus through the Internet, in which the server apparatus includes a storage unit, an extraction unit, an estimation unit, and a specifying unit, and the specifying unit specifies another user having another first information processing apparatus estimated to have been present after a date and time at which the specific first information processing apparatus was present at approximately the same position as the position estimated by the estimation unit as a contact infection possible person.

## Description

### [TECHNICAL FIELD]

The present invention relates to an information processing system, an information processing method, and a computer program.

### [BACKGROUND ART]

In a case in which a person infected with an infectious disease such as the novel coronavirus (COVID-19) or the like (hereinafter, referred to as an "infected person") is specified, in order to specify another person having a high likelihood of being infected by the infectious disease from such an infected person, it is necessary to acquire a behavior history of the infected person.

In the present situation, generally, such a behavior history is acquired by performing an interview survey for an infected person and is unreliable.

Recently, applications in which, by recording information of other persons present near someone's smartphone using a near field communication function of mutual smartphones, in a case in which he or she becomes an infected person, a close contact person is specified on the basis of this information have been developed.

Generally, the close contact person described here is a person who have had a contact with the infected person without necessary infection preventive measures at a distance at which hand contact or a face-to-face conversation is possible.

### [NON PATENT LITERATURE]

### [NON PATENT LITERATURE 11

"Singapore, Corona Infection Tracked Using Application, Developed by Government", Nihon Keizai Shinbun Electronic Edition, Retrieved on April 2, 2020

<URL:https://www.nikkei.com/article/DGXMZO57056430Q0A320C2FF8000/>

### [SUMMARY OF INVENTION]

### [TECHNICAL PROBLEM]

However, according to such a technology, information of other persons is recorded in individual smartphones, and thus there is a problem in that management performed by a team that tracks a behavior history is not easy.

Recently, examples in which an infection is checked even in the case of no direct contact with an infected person, in other words, in a case in which the person is not a close contact person, have been reported.

Thus, an object of the present disclosure is to provide an information processing system, an information processing method, and a computer program capable of easily and reliably specifying a behavior history of an infected person and extracting a person having a high likelihood of having been infected with an infectious disease from the infected person.

### [SOLUTION TO PROBLEM]

An information processing apparatus according to the present disclosure is an information processing system comprising: a second information processing apparatus having a function of receiving a signal transmitted from a first information processing apparatus; and a server apparatus that is connectable to at least the second information processing apparatus through the Internet, in which the server apparatus includes: a storage unit storing a set of at least first identification information of one or more first information processing apparatuses transmitting a signal received by the second information processing apparatus, time information at which the signal has been received, and intensity information of the signal in association with second identification information of the second information processing apparatus; an extraction unit extracting a set including time information included in specific date and time information relating to a specific date and time among sets in which specific first identification information is included in a case in which the server apparatus receives designation of the specific first identification information of the specific first information processing apparatus among one or more first information processing apparatuses and the specific date and time; an estimation unit estimating a position at which the specific first information processing apparatus was present on the basis of intensity information included in the set extracted by the extraction unit; and a specifying unit specifying another user having another first information processing apparatus estimated to have been present after a date and time at which the specific first information processing apparatus was present at approximately the same position as the position estimated by the estimation unit as a contact infection possible person.

The specifying unit can specify another first information processing apparatus estimated to have been present at approximately the same position as the position estimated by the estimation unit between the date and time at which the first information processing apparatus was present and a predetermined end date and time

The server apparatus can set the predetermined end date and time on the basis of third identification information received from a third information processing terminal and a reception time of the third identification information.

By reading a two-dimensional barcode displayed in a medium installed for each predetermined area, the third information processing terminal can transmit the third identification information to the server apparatus.

The specific date and time information can include a date and time that is a first period before the specific date and time.

The specific date and time can be configured to be a date and time at which a specific user using the specific first information processing apparatus has been diagnosed to have a predetermined infectious disease, and the first period can be configured to be an incubation period of the infectious disease.

The specific date and time information can be configured to include a date and time that is a second period after the specific date and time.

The server apparatus can be configured to further include a notification unit notifying a user having another first information processing apparatus having another first identification information specified by the specifying unit of specific information.

The notification unit can be configured to notify a user having another first information processing apparatus having another first identification information not associated with denial information among other pieces of first identification information specified by the specifying unit of specific information.

The specifying unit can be configured to specify another user having another first information processing apparatus that is estimated to have been present at the date and time at which the specific first information processing apparatus was present within a predetermined range from the position estimated by the estimation unit as a close contact person.

A plurality of second information processing apparatuses can be configured to be installed in a predetermined area, and the estimation unit can be configured to estimate a position at which the specific first information processing apparatus was present on the basis of intensity information included in a set extracted from the plurality of second information processing apparatuses.

An information processing method according to the present disclosure is an information processing method executed in an information processing system including a second information processing apparatus having a function of receiving a signal transmitted from a first information processing apparatus and a server apparatus that is connectable to at least the second information processing apparatus through the Internet, the information processing method causing the server apparatus to execute: a storage step of storing a set of at least first identification information of one or more first information processing apparatuses transmitting a signal received by the second information processing apparatus, time information at which the signal was received, and intensity information of the signal in association with second identification information of the second information processing apparatus; an extraction step of extracting a set including time information included in specific date and time information relating to a specific date and time among sets in which specific first identification information is included in a case in which the server apparatus receives designation of the specific first identification information of the specific first information processing apparatus among one or more first information processing apparatuses and the specific date and time; an estimation step of estimating a position at which the specific first information processing apparatus was present on the basis of intensity information included in the set extracted in the extraction step; and a specifying step of specifying another user having another first information processing apparatus estimated to have been present at approximately the same position as the position estimated in the estimation step after the date and time at which the specific first information processing apparatus was present as a contact infection possible person.

A computer program according to the present disclosure is a computer program executed in an information processing system including a second information processing apparatus having a function of receiving a signal transmitted from a first information processing apparatus and a server apparatus that is connectable to at least the second information processing apparatus through the Internet, the computer program causing the server apparatus to implement: a storage function of storing a set of at least first identification information of one or more first information processing apparatuses transmitting a signal received by the second information processing apparatus, time information at which the signal was received, and intensity information of the signal in association with second identification information of the second information processing apparatus; an extraction function of extracting a set including time information included in specific date and time information relating to a specific date and time among sets in which specific first identification information is included in a case in which the server apparatus receives designation of the specific first identification information of the specific first information processing apparatus among one or more first information processing apparatuses and the specific date and time; an estimation function of estimating a position at which the specific first information processing apparatus was present on the basis of intensity information included in the set extracted in the extraction function; and a specifying function of specifying another user having another first information processing apparatus estimated to have been present at approximately the same position as the position estimated in the estimation function after the date and time at which the specific first information processing apparatus was present as a contact infection possible person.

### [ADVANTAGEOUS EFFECTS OF INVENTION]

According to the present disclosure, an information processing system, an information processing method, and a computer program capable of easily and reliably specifying a behavior history of an infected person and extracting a person having a high likelihood of having been infected with an infectious disease from the infected person can be provided.

### [BRIEF DESCRIPTION OF DRAWINGS]

- **Fig. 1**: is a system configuration diagram illustrating an example of a configuration of an information processing system according to an embodiment of the present disclosure.
- **Fig. 2**: is a hardware configuration diagram illustrating a hardware configuration of a first information processing apparatus according to an embodiment of the present disclosure.
- **Fig. 3**: is a functional configuration diagram illustrating a functional configuration of the first information processing apparatus according to an embodiment of the present disclosure.
- **Fig. 4**: is a functional configuration diagram illustrating a functional configuration of a second information processing apparatus according to an embodiment of the present disclosure.
- **Fig. 5**: is a functional configuration diagram illustrating a functional configuration of a server apparatus according to an embodiment of the present disclosure.
- **Fig. 6**: is an image diagram illustrating an example of a data table stored in a storage unit of the server apparatus according to an embodiment of the present disclosure.
- **Fig. 7**: is an image diagram illustrating an image according to an embodiment of the present disclosure.
- **Fig. 8**: is a functional configuration diagram illustrating another example of a functional configuration of the server apparatus according to an embodiment of the present disclosure.
- **Fig. 9**: is an image diagram illustrating an example of a data table stored in a storage unit of the server apparatus according to an embodiment of the present disclosure.
- **Fig. 10**: is a functional configuration diagram illustrating another example of a functional configuration of a second information processing apparatus according to an embodiment of the present disclosure.
- **Fig. 11**: is a system configuration diagram illustrating an example of a configuration of the loT connection system according to an embodiment of the present disclosure.
- **Fig. 12**: is an image diagram illustrating an example of a plurality of private clouds of an loT connection system according to an embodiment of the present disclosure.
- **Fig. 13**: is an image diagram illustrating an example of a plurality of first devices connected to a private cloud of the loT connection system according to an embodiment of the present disclosure.
- **Fig. 14**: is an image diagram illustrating an example of the flow of a service provided by the loT connection system according to an embodiment of the present disclosure.
- **Fig. 15**: is a hardware configuration diagram illustrating a hardware configuration of an IoT router according to an embodiment of the present disclosure.
- **Fig. 16**: is an image diagram illustrating an example of the flow of a service provided by the IoT connection system according to an embodiment of the present disclosure.
- **Fig. 17**: is an image diagram for describing a connection interface of the loT connection system according to an embodiment of the present disclosure.
- **Fig. 18**: is an image diagram illustrating another example of the flow of a service provided by the loT connection system according to an embodiment of the present disclosure.
- **Fig. 19**: is an image diagram illustrating an example of the flow of a virtual driver function provided by the loT connection system according to an embodiment of the present disclosure.
- **Fig. 20**: is an image diagram illustrating another example of the flow of a virtual driver function provided by the loT connection system according to an embodiment of the present disclosure.
- **Fig. 21**: is a flowchart illustrating an example of the flow of an information processing method according to an embodiment of the present disclosure.
- **Fig. 22**: is a conceptual diagram illustrating another example of the configuration of the information processing system according to an embodiment of the present disclosure.

### [DESCRIPTION OF EMBODIMENTS]

An embodiment of an information processing system of the present disclosure will be described with reference to the drawings.

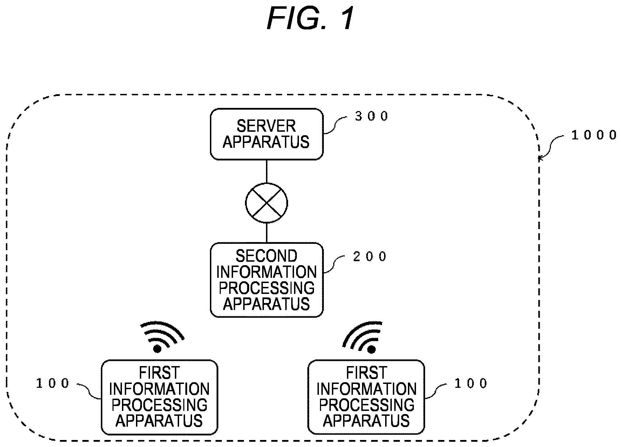

As illustrated in FIG. 1 as an example, an information processing system 1000 according to the present disclosure includes a second information processing apparatus 2-0 having a function of receiving a signal transmitted from a first information processing apparatus 100 and a server apparatus 300 that can be connected to at least the second information processing apparatus 200 through the Internet.

The first information processing apparatus 100 is a device having a function of transmitting signals.

The function of transmitting signals is a function enabling transmission of information to a device that can receive signals, for example, using a radio technology such as Bluetooth (registered trademark) Low Energy (BLE), Wi-Fi, or the like.

Although the first information processing apparatus 100 is not particularly limited as long as it is a device having the function of transmitting signals described above, for example, it may be an information processing apparatus such as a smartphone, a tablet, or the like.

The function of transmitting the signals described above can be realized by the first information processing apparatus 100 by installing a dedicated application.

The second information processing apparatus 200 is a device having a function of receiving the signals described above.

Although the second information processing apparatus 200 is not particularly limited as long as it is a device having the function of receiving a signal described above, for example, it may be an information processing apparatus such as a smartphone, a tablet, or the like. In addition, as the second information processing apparatus 200, an loT router to be described below may be used.

The function of receiving the signals described above can be realized by the second information processing apparatus 200 by installing a dedicated application.

The server apparatus 300 is an apparatus that can be connected to the second information processing apparatus 200 through the Internet.

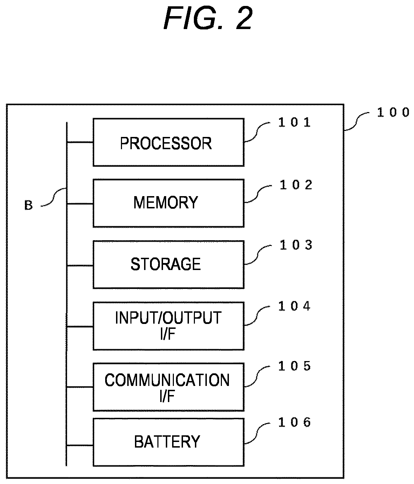

Here, the hardware configuration of the first information processing apparatus 100 will be described with reference to FIG. 2. The first information processing apparatus 100 may include a processor 101, a memory 102, a storage 103, an input/output interface (input/output I/F) 104, and a communication interface (communication I/F) 105. The constituent elements are connected to each other through a bus B.

Through cooperation among the processor 101, the memory 102, the storage 103, the input/output I/F 104, and the communication I/F 105, the first information processing apparatus 100 can realize functions and methods described in this embodiment.

The processor 101 executes functions and/or methods realized using codes or commands included in a program stored in the storage 103. The processor 201, for example, includes a central processing unit (CPU), a micro processing unit (MPU), a graphics processing unit (GPU), a microprocessor, a processor core, a multiprocessor, an application-specific integrated circuit (ASIC), a field programmable gate array (FPGA), and the like and may realize each process disclosed in each embodiment using logic circuits (hardware) and dedicated circuits formed in an integrated circuit (an integrated circuit (IC) chip or a large scale integration (LSI)) or the like. In addition, such circuits may be realized using one or a plurality of integrated circuits, and a plurality of processes represented in each embodiment may be realized using one integrated circuit. The LSI may also be referred to as a VLSI, a super LSI, an ultra LSI, or the like in accordance with a difference of the degree of integration.

The memory 102 temporarily stores a program loaded from the storage 103 and provides a work area for the processor 101. In the memory 102, various kinds of data generated while the processor 101 executes a program are also temporarily stored. The memory 102, for example, includes a random access memory (RAM), a read only memory (ROM), and the like.

The storage 103 stores a program. The storage 103, for example, includes a hard disk drive (HDD), a solid state drive (SSD), a flash memory, and the like.

The communication I/F 105 is implemented as hardware such as a network adaptor or the like, communication software, and a combination thereof and transmits and receives various kinds of data through a network. This communication may be performed either in a wired manner or in a wireless manner, and any communication protocol may be used as long as mutual communication can be performed. The communication I/F 105 performs communication with other information processing apparatuses through a network. The communication I/F 105 transmits various kinds of data to other information processing apparatuses in accordance with an instruction from the processor 101. In addition, the communication I/F 105 receives various kinds of data transmitted from other information processing apparatuses and transfers the data to the processor 101.

The input/output I/F 104 includes an input device that inputs various kinds of operations for the first information processing apparatus 100 and an output device that outputs a process result processed by the first information processing apparatus 100. In the input/output I/F 104, the input device and the output device may be integrated, or the input device and the output device may be separated from each other.

The input device receives an input from a user and is realized using any kind of device that can transfer information relating to the input to the processor 101 or a combination of such devices. The input device, for example, includes a hardware key such as a touch panel, a touch display, a keyboard, or the like, a pointing device such as a mouse or the like, a camera (an operation input through an image), and a microphone (an operation input using a voice).

The output device outputs a process result processed by the processor 101. The output device, for example, includes a touch panel, a speaker, and the like.

A hardware configuration similar to that of the first information processing apparatus 100 described above may also be employed for the second information processing apparatus 200 and the server apparatus 300 described above.

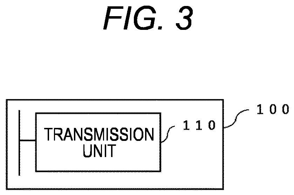

As illustrated in FIG. 3, the first information processing apparatus 100 of the present disclosure may include a transmission unit 110.

The transmission unit 110 transmits a signal including first identification information of the first information processing apparatus 100.

The first identification information is not particularly limited, as long as it is identification information that can be used for specifying the first information processing apparatus 100. Although a unique device identifier (UDID), a universally unique identifier (UUID), and the like can be used as examples of the first identification information, it is preferable that the UUID be set as the first identification information from the point of view of privacy and security.

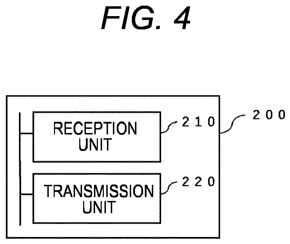

As illustrated in FIG. 4, the second information processing apparatus 200 may include a reception unit 210 and a transmission unit 220.

The reception unit 210 receives a signal transmitted from the first information processing apparatus 100.

The transmission unit 220 transmits time information, intensity information, and first identification information to the server apparatus 300 together with second identification information of the second information processing apparatus 200.

The time information is information relating to a time at which the signal described above is received. Such time information includes a date and a time, and the time may include hours, minutes, and seconds.

The intensity information is information based on a reception intensity of a signal. The information relating to a reception intensity may include a reference intensity in addition to an electric wave intensity representing an intensity of a received electric wave. An estimated distance between the first information processing apparatus 100 and the second information processing apparatus 200 may be calculated from these, and this estimated distance may be set as information based on the reception intensity. Such calculation of an estimated distance may be performed by either the second information processing apparatus 200 or the server apparatus 300.

The second identification information is not particularly limited as long as the information is identification information that can be used for specifying the second information processing apparatus 200. Similar to the first identification information, as examples of the second identification information, although a unique device identifier (UDID), a universally unique identifier (UUID), and the like can be used, it is preferable that the UUID be set as the second identification information from a point of view of privacy and security. This UUID is a numerical value issued by the dedicated application described above and may be configured not to include personal information.

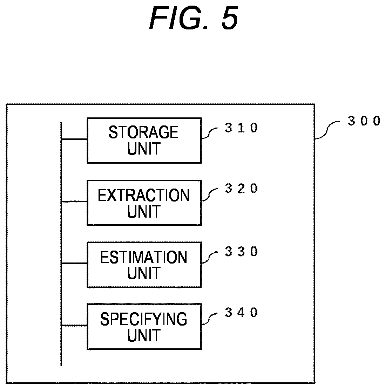

As illustrated in FIG. 5, the server apparatus includes a storage unit 310, an extraction unit 320, an estimation unit 330, and a specifying unit 340.

The storage unit 310 stores a set of at least first identification information of one or more first information processing apparatuses 100 transmitting a signal received by the second information processing apparatus 200, time information at which the signal has been received, and intensity information of the signal in association with the second identification information of the second information processing apparatus 200.

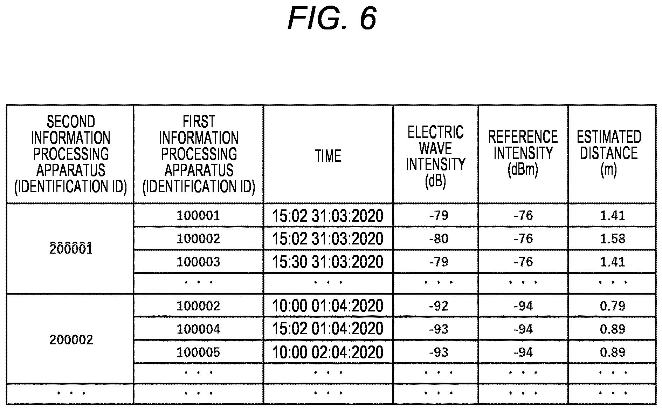

FIG. 6 illustrates an example of a data table stored by the storage unit 310. As illustrated in FIG. 6, an identification ID of the first information processing apparatus 100 that has transmitted the received signal, a reception time, and intensity information are associated with an identification ID of the second information processing apparatus 200.

In FIG. 6, although only one piece of time information is represented in each piece of the first identification information, the time information may be stored with a predetermined interval from a time at which a signal is received for the first time to a time at which the signal is not received.

In a case in which the server apparatus 300 receives designation of specific first identification information and a specific date and time of a specific first information processing apparatus among one or more first information processing apparatuses 100, the extraction unit 320 extracts a set including time information included in specific date and time information relating to the specific date and time among sets in which the specific first identification information is included.

More specifically, the accepting unit 320 receives designation of specific first identification information of a specific first information processing apparatus 100A. This specific first information processing apparatus 100A may be a mobile terminal such as a smartphone or the like owned by an infected person (a person diagnosed with an infectious disease through a test; hereinafter, referred to as a "specific user").

The designation described above is performed through a predetermined information processing terminal by a user using the information processing system 1000 according to the present disclosure.

In this case, examples of a user performing the designation described above include persons tracking a behavior history of an infected person, for example, staff of a health care center, medical professionals, and the like but are not limited to such persons.

For example, a specific user who is an infected person may transmit information indicating a diagnosis of an infectious disease from his or her own first information processing apparatus 100 to the server apparatus 300 and designate specific first identification information such that his or her own first information processing apparatus 100 is the specific first information processing apparatus 100A.

The specific date and time may be a date and time at which a specific user has been diagnosed to have an infectious disease through a test. In addition, basically, in a case in which a certain symptom appears, a user takes a test for an infectious disease by having a check-up with a medical organization, and thus a date and time at which this infectious disease is diagnosed can also be regarded as an outbreak date of the infectious disease. In a case in which only a date is included in a specific date and time, hours and minutes may not be included.

In addition, the specific date and time information relating to a specific date and time can be configured to include a period from a date and time that is a first period before the specific date and time to the specific date and time. This first period can be set as an incubation period of an infectious disease and can be appropriately set in accordance with a kind of infectious disease. For example, the first period is set as two days.

Then, in order to specify in which place of which room a specific user was present in a first period with the incubation period taken into account on the basis of an outbreak date of an infectious disease of the specific user, the extraction unit 320 extracts a set including time information included in the first period among sets including specific first identification information stored in the storage unit 310.

The estimation unit 330 estimates a position at which the specific first information processing apparatus 100A was present on the basis of intensity information included in the set extracted by the extraction unit 320.

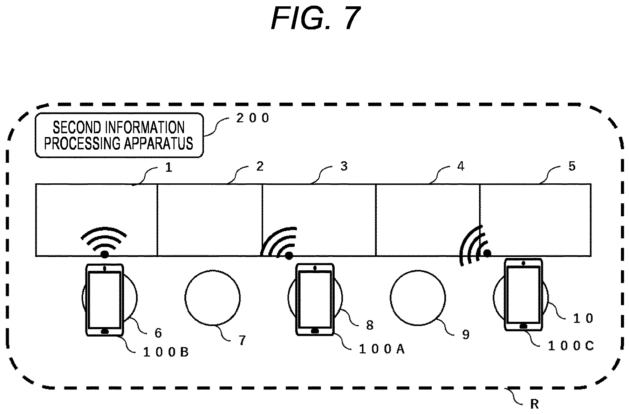

FIG. 7 is an image diagram assuming a state in which a specific user having a specific first information processing apparatus 100A is sitting on a chair 8 corresponding to a desk 3 in a predetermined area R in which desks 1 to 5 and chairs 6 to 10 are disposed as an example.

In addition, as an infection prevention countermeasure, it is preferable that partition plates be disposed between the desks 1 to 5.

In such a state, there is a likelihood of the specific user spreading viruses to the surroundings by touching the desk 3 or the chair 8 with his or her hand to which the viruses are attached, coughing, sneezing, or the like.

There is a likelihood of viruses spread to the surroundings infecting users having a first information processing terminal 100B or a first information processing terminal 100C who were present in the same area R at the same timing as the specific user (specifying of close contact persons will be described below), and viruses attached to the desk 3 and the chair 8 have a likelihood of infecting users who touched the desk 3 and the chair 8 even after the specific user is gone.

In the predetermined area R, one or more second information processing apparatuses 200 are disposed, and the second information processing apparatuses 200 receive signals from one or more first information processing apparatuses 100.

In a case in which an electric wave intensity is stored in the storage unit 310 as intensity information, an estimated distance is calculated on the basis of a reference intensity measured at a reference distance in advance, and a position at which the specific first information processing apparatus 100A was present is estimated. In addition, in a case in which an estimated distance that has already been calculated is stored in the storage unit 310, a position at which a specific first information processing apparatus 100A was present is estimated on the basis of such a stored estimated distance.

Then, the specifying unit 340 specifies another user having another first information processing apparatus 100D estimated to have been present after a date and time at which the specific first information processing apparatus 100A was present at approximately the same position as the position estimated by the estimation unit 330 as a person for whom contact infection is possible.

For example, the position that is approximately the same as a position estimated by the estimation unit 330 may be set as a range in which viruses are attached in accordance with a specific user sitting on the chair 8 touching with a hand to which viruses are attached and/or coughing, sneezing, or the like, that is, a position at which the user is estimated to sit on the chair 8.

According to the configuration described above, technical improvement for solving or alleviating at least some of the problems of the conventional technology described above can be provided. More specifically, according to the present disclosure, specifying a behavior history of an infected person and extracting a person having a high likelihood of having been infected with an infectious disease from an infected person can be performed easily and reliably.

In addition, the information processing system according to the present disclosure can estimate in which place of which room a specific user was present in a period with an incubation period taken into account on the basis of an outbreak date of an infectious disease of the specific user and, thereafter, can specify another user (a person for whom contact infection is possible) estimated to have been present at the same place of the same room.

In addition, when the second information processing apparatus 200 is installed in each room in an office, in a case in which there is an infected person, information such as a degree of time for which the infected person stayed in a certain room, who was a close contact person or for whom contact infection is possible, and the like can be quickly extracted.

By quickly specifying close contact persons or people for whom contact infection is possible, effective countermeasures such as causing only the close contact persons or the people for whom contact infection is possible to stay at home or the like, rather than all the people who entered and exited the office, can be quickly taken. As a result, a situation in which the entire office is closed can be avoided, and the countermeasure also becomes a business continuation plan (BCP) countermeasure.

In addition, a plurality of second information processing apparatuses 200 may be installed in a predetermined area.

In such a case, the estimation unit 330 can estimate a position at which the specific first information processing apparatus was present on the basis of intensity information included in a set extracted from the plurality of second information processing apparatuses 200. As a technology of such estimation, a known technology can be used.

According to the configuration described above, accuracy of estimation of a position can be improved.

The specifying unit 340 can specify another first information processing apparatus estimated to have been present at approximately the same position as the position estimated by the estimation unit 330 between a date and time at which the first information processing apparatus was present and a predetermined end date and time.

In other words, the predetermined end date and time is a date and time at which attached viruses have died. A period in which viruses automatically died after attachment of the viruses can be appropriately set on the basis of a kind of viruses, a material of an attachment object, and the like. For example, it is regarded that it takes about two days until coronaviruses attached to a wooden desk die.

Alternatively, the predetermined end date and time is a date and time at which an attachment object having a likelihood of viruses being attached thereto is sterilized. In a case in which sterilization is performed with each use, a period until sterilization after attachment of viruses is different in accordance with a case in which sterilization is performed at the time of cleaning and the like.

In this way, in a case in which viruses die, or an attachment object is sterilized, there is a low likelihood of infection even when a person is present at the place thereafter, and thus specifying of a user after a predetermined end date and time becomes unnecessary.

The server apparatus 300 can set the predetermined end date and time on the basis of third identification information received from the third information processing terminal and a reception time of the third identification information.

For example, the third information processing terminal is assumed to be used by cleaner performing sterilization of a desk, a chair, and the like. According to this, in a case in which the second information processing apparatus 200 installed in a predetermined area R receives third identification information transmitted from the third information processing terminal held by the cleaner, a predetermined end date and time can be set for sterilization of desks and chairs (elimination of viruses).

Alternatively, by reading a two-dimensional barcode displayed in a medium installed for each predetermined area, the third information processing terminal can transmit the third identification information to the server apparatus 300.

For example, the two-dimensional barcode is a QR code (registered trademark), and the medium may be a seal that can be attached to a desk.

In this way, in accordance with reception of the third identification information, data of a time series stored in association with the second identification information can be sealed with a timestamp.

In addition, the specific date and time information may include a date and time that is a second period after the specific date and time.

In a case in which the specific date and time is a date and time at which an infectious disease was diagnosed, although it is assumed that the infected person does not go out (commute to the office or the like) thereafter, in a case in which the specific date and time is a date and time at which the symptoms appeared, and thereafter, the person commutes to the office or the like until the diagnosis, a period (a second period) from a specific date and time to a final commute date and time needs to be included in the specific date and time information.

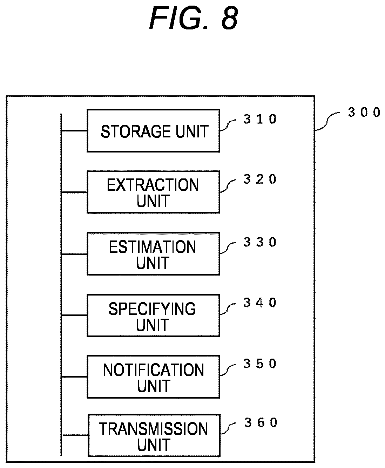

As illustrated in FIG. 8, the server apparatus 300 may further include a notification unit 350.

The notification unit 350 notifies a user having another first information processing apparatus having other first identification information specified by the specifying unit 340 of specific information.

For example, the specific information may be information of details indicating a person for whom contact infection is possible. In addition, information of a nearby facility that can handle an infectious disease and information of a telephone number of a help desk and the like may be included in such information.

It is preferable that the notification be forcibly performed using a technology of a push notification.

The notification unit 350 can notify a user having another first information processing apparatus having other first identification information not associated with denial information among other first identification information specified by the specifying unit 340 of specific information.

The denial information is registered by a user and, for example, is information indicating that a countermeasure for prevention of an infection according to contact with an attachment object has been performed such as the user having sterilized a desk 3 and a chair 8 before sitting on the chair 8, the user having washed his or her hands after leaving the seat, or the like.

Although the embodiment of the invention for specifying a person for whom contact infection is possible person who had no direct contact with an infected person has been described above, the information processing system according to the present disclosure can specify a close contact person as well.

More specifically, the specifying unit 340 can specify another user having another first information processing apparatus that is estimated to have been present at a date and time at which the specific first information processing apparatus 100A was present within a predetermined range from a position estimated by the estimation unit 330 as a close contact person.

The other user specified as this close contact person is a user having the first information processing apparatus 100B or the first information processing apparatus 100C illustrated in FIG. 7.

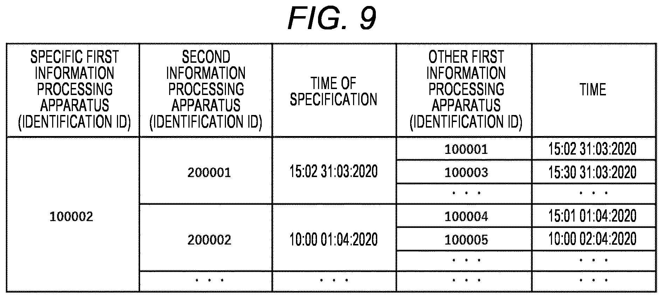

An example representing an image of another first identification information extracted in a case in which the specific first identification information of the specific first information processing apparatus 100A is "100002" illustrated in FIG. 6 is illustrated in FIG. 9.

For example, the date and time at which the specific first information processing apparatus 100A was present may be all the times at which a date and time is stored at a predetermined interval from a time when a signal is received from the specific first information processing apparatus for the first time to a time when no signal is received therefrom.

Alternatively, as another example, the date and time at which the specific first information processing apparatus 100A was present may include a staying time calculated on the basis of a time at which a signal was received from the specific first information processing apparatus for the first time and a time interval from a time when the signal was received for the first time to a time when no signal has been received.

In addition, the extraction unit 320 may extract another second identification information on the basis of a filter condition based on the intensity information.

In other words, in a case in which an infected person is estimated to be present in a room in which the second information processing apparatus 200 is installed, persons estimated to be present outside the room on the basis of the intensity information may not be extracted as close contact persons.

The notification unit 330 notifies the extracted other first information processing apparatuses of specific information.

For example, the specific information may be information of details indicating being a close contact person. In such information, information of a nearby facility that can handle the infectious disease and information of a telephone number of a help desk and the like may be included.

It is preferable that the notification be forcedly performed using a technology of a push notification.

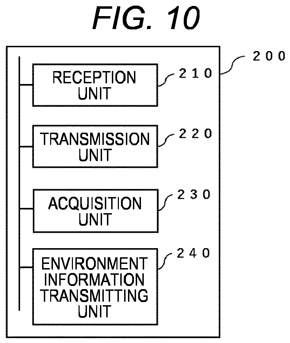

In addition, as illustrated in FIG. 10, the second information processing apparatus 200 may further include an acquisition unit 230 and an environment information transmitting unit 240.

The acquisition unit 230 acquires environment information of an installation place from a predetermined sensor.

The predetermined sensor is not particularly limited as long as it can measure environment information of an installation place and, for example, can be configured to be at least one of a room temperature sensor, a humidity sensor, an illuminance sensor, an atmospheric pressure sensor, a noise sensor, various gas density sensors, a discomfort index sensor, a heat stroke degree of warning sensor, and the like.

Examples of a gas that can be measured by a gas sensor include CO2, a volatile organic solvent quantity ray, and the like.

The environment information transmitting unit 240 transmits time information relating to a time at which the environment information was received to the server apparatus 300 together with the environment information.

Then, the storage unit 310 of the server apparatus 300 can store the environment information and the time information received from the second information processing apparatus 200 in association with the second identification information.

Then, in a case in which environment information received from the second information processing apparatus 200 satisfies a specific condition, the extraction unit 330 of the server apparatus 300 may extract one or more pieces of first identification information stored in association with the second identification information.

The specific condition may be set as a condition satisfying conditions of so-called "three closings" (closing/close formation/closeness). Specific numerical values may be appropriately set in advance.

In addition, the notification unit 350 described above can notify at least one of first information processing apparatuses 100 having one or more pieces of first identification information extracted in a case in which the specific condition is satisfied of attention information.

In addition, in a case in which a room satisfies the condition of "three closings", attention information urging air ventilation or the like can be notified to an organizer of a conference or the like.

Alternatively, in a case in which the environment information satisfies the specific condition, "three closings" may be resolved by automatically controlling indoor loT devices (an air conditioner, an air cleaner, a humidifier, and the like). Automatic control of loT devices can be applied to a case in which the second information processing apparatus 200 is an loT router as will be described below.

The second information processing apparatus 200 according to the present disclosure can be configured to be an loT router.

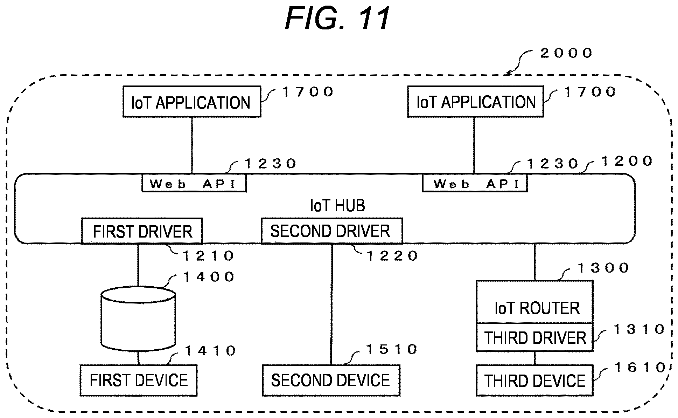

As illustrated in FIG. 11, the loT router is an loT router 1300 in an IoT connection system 2000 including an loT hub 1200 realized on a cloud and an loT router 1300 locally connected to the loT hub 1200.

Here, an embodiment of the loT connection system 2000 according to an embodiment of the present disclosure will be described with reference to the drawings.

As illustrated in FIG. 11, the loT connection system 2000 according to an embodiment of the present disclosure includes an IoT hub 1200 and an IoT router 1300.

The loT hub 1200 is configured to be realized on a cloud. More specifically, the loT hub 1200 is a managed service hosted inside a cloud and functions as a repeater for bidirectional communication between an loT application (hereinafter, referred to as "loT application") and an loT device.

The loT router 1300 is locally connected to the loT hub 1200 through a wide area network (WAN).

More specifically, the loT router 1300 realizes an loT device not connected to the Internet such as a home network or the like being connected to the loT hub 1200.

The loT hub 1200 includes at least one of a first driver 1210 or a second driver 1220.

The first driver 1210 and the second driver 1220 absorb differences in specifications for respective manufacturers of IoT devices.

The first driver 1210 is used for connecting a private cloud 1400 to which the first device 1410 can be connected and the loT hub 1200.

For example, it is preferable that the first device 1410 and the private cloud 1400 are connected through a local area network (LAN) or a WAN, and the private cloud 1400 and the first driver 1210 be connected also through a LAN or a WAN.

The private cloud 1400 is provided by a service provider of the first device 1410. In FIG. 11, although a case in which there is one private cloud 1400 is illustrated, the number of private clouds is not limited to one, and a plurality of private clouds 1400 can be configured to be connected to the loT hub 1200. In addition, the loT hub 1200 may include a plurality of first drivers 1210.

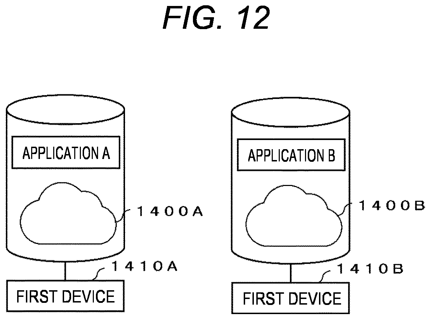

FIG. 12 illustrates details of two private clouds 1400A and 1400B provided by different service providers A and B. As illustrated in FIG. 12, the private cloud 1400A is connected to an application A (hereinafter referred to as "application A") provided by the service provider A and provides a service according to the application A for the first device 1410.

Similarly, the private cloud 1400B is connected to an application B (hereinafter referred to as "application B") provided by the service provider B and provides a service according to the application B for the first device 1410.

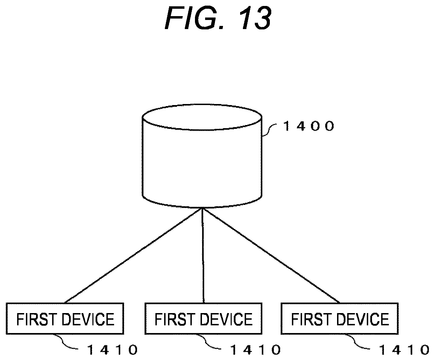

In addition, in FIGS. 11 and 12, although an example in which only one first device 1410 is connected to the private cloud 1400 is illustrated, as illustrated in FIG. 13, a plurality of first devices 1410 may be connected to one private cloud 1400.

The first device 1410 can be configured as a device for which a service provider provides a private cloud. Examples thereof may be an electronic lock having a remote locking function, an AI speaker, a nursing bed that can be remotely operated, and the like, but the first device is not particularly limited thereto.

Referring to FIG. 11, the second driver 1220 is used for directly connecting the second device 1510 and the loT hub 1200.

The second device 1510 can be connected to the loT hub 1200 present on the Internet through a WAN (may be through a LAN).

In FIG. 11, although an example in which only one second device 1510 is connected to the second driver 1220 is illustrated, a plurality of second devices 1510 may be connected to one second driver 1220. In addition, the loT hub 1200 may have a plurality of second drivers 1220.

The second device 1510 may be a device for which a service provider does not provide a private cloud. Examples thereof include an electric fan, an air conditioner, a window, a curtain, a lamp, and the like, but the second device is not particularly limited thereto.

The loT router 1300 has a third driver 1310. In addition, the loT router 1300 may have a plurality of third drivers 1310.

The third driver 1310 is used for connecting the third device 1610 and the loT router 1300.

For example, it is preferable that the third device 1610 and the third driver 1310 be connected through a LAN, and the loT router 1300 and the loT hub 1200 be connected through a WAN.

As described above, the third device 1610 can be configured to be an loT device not connected to the Internet such as a home network. In addition, from the point of view of security, privacy, and safety, the third device 610 can be configured to be a device that cannot be directly connected to the loT hub 1200. Examples thereof include a gas stove, a face authentication device, a data logger for collecting sensor information and the like, but the third device is not particularly limited thereto. However, from the point of view of reduction of risk at the time of a disaster to be described below, even such a device may be connected to the loT router 1300 as the third device 1610.

In this way, the loT connection system 2000 according to the present invention is an loT connection system of a hybrid type in which not all the devices are directly connected to the loT hub 1200 on the cloud, but some devices are locally connected to the loT router 1300.

In this way described above, not only loT devices that are directly connected but loT devices connected to conventional private clouds can be easily connected to each other.

In accordance with this, different from a conventional case in which only loT devices of set manufacturers are connected, loT devices of various manufacturers can be easily connected to each other. In addition, by connecting loT devices of various manufacturers to each other, a unique service that is not conventionally present can be created.

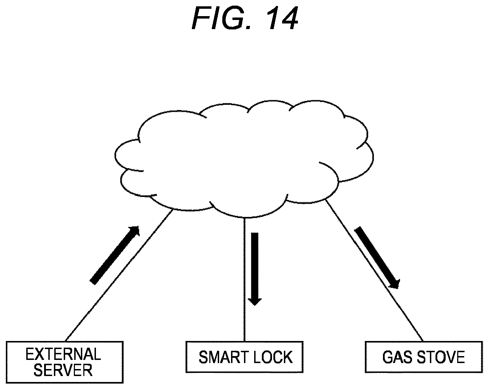

For example, according to the loT connection system 2000 of the present invention, as illustrated in FIG. 14, a service for transmitting an extinction signal to a gas stove and unlocking a key of an entrance door at the time of receiving an urgent earthquake warning from an external server can be easily realized.

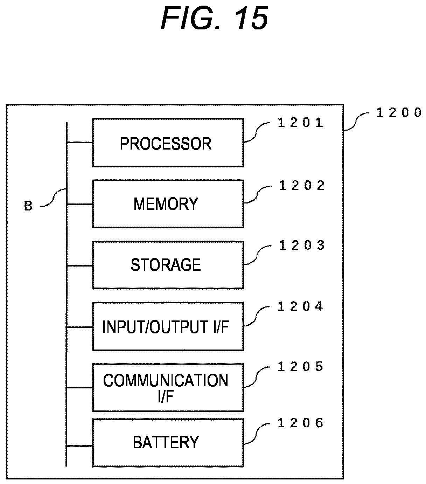

Hereinafter, a hardware configuration of the information processing terminal (the loT hub) 1200 will be described with reference to FIG. 15. The information processing terminal 1200 may include a processor 1201, a memory 1202, a storage 1203, an input/output interface (input/output I/F) 1204, and a communication interface (communication I/F) 1205. The constituent elements are connected to each other through a bus B.

In accordance with the processor 1201, the memory 1202, the storage 1203, the input/output I/F 1204, and the communication I/F 1205 in cooperation, the information processing terminal 1200 can realize functions and methods described in this embodiment.

The processor 1201 executes functions and/or methods realized using codes or commands included in a program stored in the storage 1203. The processor 1201, for example, includes a central processing unit (CPU), a micro processing unit (MPU), a graphics processing unit (GPU), a microprocessor, a processor core, a multiprocessor, an application-specific integrated circuit (ASIC), a field programmable gate array (FPGA), and the like and may realize each process disclosed in each embodiment using logic circuits (hardware) and dedicated circuits formed in an integrated circuit (an integrated circuit (IC) chip or a large scale integration (LSI)) or the like. In addition, such circuits may be realized using one or a plurality of integrated circuits, and a plurality of processes represented in each embodiment may be realized using one integrated circuit. The LSI may also be referred to as a VLSI, a super LSI, an ultra LSI, or the like in accordance with a difference of the degree of integration.

The memory 1202 temporarily stores a program loaded from the storage 1203 and provides a work area for the processor 1201. In the memory 1202, various kinds of data generated while the processor 1201 executes a program are also temporarily stored. The memory 1202, for example, includes a random access memory (RAM), a read only memory (ROM), and the like.

The storage 1203 stores a program. The storage 1203, for example, includes a hard disk drive (HDD), a solid state drive (SSD), a flash memory, and the like.

The communication I/F 1205 is implemented as hardware such as a network adaptor or the like, communication software, and a combination thereof and transmits and receives various kinds of data through a network. This communication may be performed either in a wired manner or in a wireless manner, and any communication protocol may be used as long as mutual communication can be performed. The communication I/F 1205 performs communication with other information processing apparatuses through a network. The communication I/F 1205 transmits various kinds of data to other information processing apparatuses in accordance with an instruction from the processor 1201. In addition, the communication I/F 1205 receives various kinds of data transmitted from other information processing apparatuses and transfers the data to the processor 1201.

The input/output I/F 1204 includes an input device that inputs various kinds of operations for the information processing terminal 1200 and an output device that outputs a process result processed by the information processing terminal 1200. In the input/output I/F 1304, the input device and the output device may be integrated, or the input device and the output device may be separated from each other.

The input device receives an input from a user and is realized using one of all kinds of devices that can transfer information relating to the input to the processor 1201 or a combination thereof. The input device, for example, includes a hardware key such as a touch panel, a touch display, a keyboard, or the like, a pointing device such as a mouse or the like, a camera (an operation input through an image), and a microphone (an operation input using a voice).

The output device outputs a process result processed by the processor 1201. The output device, for example, includes a touch panel, a speaker, and the like.

A hardware configuration similar to that of the loT hub 1200 described above can be employed also for the loT router 1300 described above.

In addition, it is preferable that the loT router 1300 be an information processing terminal that is able to communicate with a predetermined base station.

For example, an information processing terminal that is able to communicate with a predetermined base station is an information processing terminal such as a smartphone, a tablet terminal, or the like that is used by inserting a SIM card thereinto. A communication capacity between the loT router 1300 and the loT hub 1200 is a low capacity that is 100 Mbytes or less per month, and thus insertion of a low-priced SIM card having a small communication capacity into the loT router 1300 is sufficient. For example, in a case in which 10 signals (persons) are present in the vicinity of one conference room on the average, and information is transmitted to a server in units of 5 minutes, a required communication capacity is 17.3 M/conference room (month).

In addition, the loT router 1300 includes a battery. Such a battery can save an electric power through charging. The loT router 1300 can realize a function according to the present disclosure for about 3 days from full-charging without being connected to a power supply. At a normal time (in a case not under a specific situation to be described below), the loT router 1300 is used with being connected to a power supply.

Then, under the specific situation, the third device 1610 can be connected to the loT hub 1200 through a tethering function of the loT router 1300.

The specific situation can be configured to be a situation in which connection between the third device 1610 and a predetermined wireless/wired LAN is cut off, a situation in which connection between the loT router 1300 and a predetermined wireless/wired LAN is cut off, or the like but is not limited thereto.

Although an example of a factor causing this specific situation may be a power failure, but the factor is not limited thereto and may be a breakdown of a Wi-Fi (registered trademark) router, a connection defect, or the like.

In addition, as the tethering function, a known technology can be used, and techniques such as Wi-Fi tethering, Bluetooth (registered trademark) tethering, USB tethering, and the like may be used.

According to the configuration described above, loTs in the life that are in a silo state for each private cloud can be freely connected to each other through the Internet, whereby an attractive service can be provided.

More specifically, according to the present disclosure, not only loT devices that are directly connected but also loT devices connected to conventional private clouds can be easily connected to each other.

In addition, according to the configuration described above, by connecting the third device 1610 to the loT hub 1200 using the tethering function of the loT router 1300 in a specific situation, even in a state in which an loT device cannot be connected to a network in a disaster or the like, a service can be continuously used.

In addition, it is preferable that the loT router 1300 be remotely controlled (remotely operated) by a manager device. According to such a configuration, each loT router can be fully supported.

In addition, the remote operation described above is realized by installing a dedicated application in both the manager device and the loT router 1300. Furthermore, in consideration of easy support of the remote operation, it is preferable that an Android terminal be used as the information processing terminal as the loT router 1300.

In addition, it is preferable that connected device management (CDM; various device managements) be performed for the loT router 1300 by a manager device. Such CDM can be realized using a known mobile device management (MDM) technique. According to such a configuration, loT routers installed in a huge number of households are universally managed instantly and can be remotely monitored and updated. In addition, according to the CDM, setting of a device and distribution and update of an application can be remotely monitored and controlled. In accordance with this, reduction of costs and operation efficiency can be realized.

In addition, although only authenticated applications and devices can be connected to the loT hub 1200, the loT router 1300 extends the loT hub 1200 to local places (homes and the like), and a real time property that can be realized only in local places, securement of security and privacy, reduction of an amount of communication, and the like can be handled.

In addition, by configuring the loT router 1300 to be an information processing terminal, a customer' smartphone, and an Internet line inside a house do not need to be used, and a risk of occurrences of unexpected troubles can be reduced.

Furthermore, in the loT connection system 2000 according to the present invention, information to be described by a user for generating the first driver 1210, the second driver 1220, and the third driver 1310 may be limited to information relating to definitions of devices and definitions of commands.

Here, a method for generating the first driver 1210, the second driver 1220, and the third driver 1310 will be described. A generating person may be a user relating to manufacturing and development of loT devices or a user relating to provision of the loT connection system according to the present invention.

In addition, program languages of the first driver 1210, the second driver 1220, and the third driver 1310 may be different from each other, as long as information of the same details is described.

Initially, a generating person defines a use device list as definitions of devices. For example, in a case in which "weather sensor", "indoor sensor", "outdoor sensor", "suspicious person sensor", "approval sensor", and "power sensor" are defined as use devices, "weather", "inhouse", "outdoor", "security", "approve", and "power" that are names and IDs thereof are described.

Subsequently, the generating person defines usable command as command definitions. For example, in a case in which a usable command of the "outdoor sensor" is defined, observation commands for a sensor value is described. For example, the observation commands can be configured to be "observe", "get", "observe an outdoor place", and the like.

In accordance with the generating person embedding information about the device definitions and the command definitions described above in a program in a filling-in a hole form, the first driver 1210, the second driver 1220, and the third driver 1310 can be completed. The other parts can be provided as an SDK from a provider.

The SDK part includes a part relating to a device operation process in a received command, a part relating to a pre-process of collected sensor data/operation result data, and a part relating to a data transmission process for the loT hub.

According to the configuration described above, drivers for loT devices of various forms can be completed in a simple manner, and thus an loT connection system capable of connecting IoT devices flexibly and easily can be realized.

Generally, acquired technical fields of engineers relating to development of devices and engineers of web development are different from each other, and there are many persons not having a technical level capable of connecting a device to an loT hub.

For this reason, as in the present disclosure, it is very advantageous to be able to generate any driver program using a simple technique of a common filling-in a hole form. In accordance with this, a development cost and a development period relating to connection of an loT device to an IoT hub can be reduced more than those of a conventional case.

In addition, in accordance with reduction of the development cost, even in a case in which there is a difference in an allowed value of the applied cost as in the case of an electric fan and an air conditioner, connection to an IoT hub can be equally realized.

Subsequently, connection between the loT hub 1200 relating to the loT connection system 2000 according to the present disclosure and an loT application will be described.

As illustrated in FIG. 11, the loT hub 1200 can have a WebAPI 1230 used for using the loT application 1700. In addition, as illustrated in FIG. 11, loT applications 1700 corresponding to the number of services can be connected, and each thereof can be connected using the WebAPI 1230.

The IoT application 1700 can be generated by describing a data acquisition logic from an loT device (sensor) and/or an IoT device operating logic inside the application.

The data acquisition logic is composed of a part that performs a pre-process of acquired sensor data and a part that performs transmission to an API of the loT hub 1200.

Necessary information is a connection destination URL provided by an operator of the loT connection system 2000 and an API key, device information, and an execution command provided by an operator.

The device operating logic is composed of a part that performs a pre-process of a device command desired to be operated and a part that performs transmission to an API of the loT hub 1200. Necessary information is a connection destination URL provided by an operator of the loT connection system 2000 and an API key, device information, and, an execution command provided by an operator.

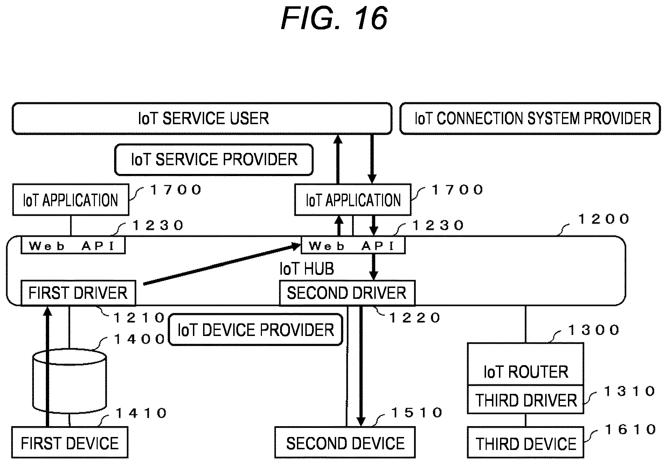

FIG. 16 is a diagram illustrating a flow in which an IoT service user (a final user) receives an loT service from an IoT service provider using an IoT device provided by an loT device provider. As illustrated in FIG. 16, first, when there is a notification of an event from the first device 1410, the event is notified to the final user through the private cloud 1400, the first driver 1210, the WebAPI 1230, and the loT application 1700.

Subsequently, when the final user determines an action, the second device 510 is instructed to execute the action through the IoT application 1700, the WebAPI 1230, and the second driver 1220.

A linkage of the loT device can be represented as an event-driven program of "when XX becomes as such, OO is performed". Then, this process is configured as components as a micro-service and configured to be common and used and can be implemented as a function of a FaaS (Function as a Service).

Meanwhile, the first device to the third device that are the loT devices described above are developed and manufactured by various manufacturers in accordance with recent development of an loT field. However, there is a problem in that constant connection with a device or an IoT application is limited to a device that can be connected to a server used for a push notification service provided by a big-company vendor such as Google LLC, Apple Inc., or the like.

The loT hub 1200 according to the present disclosure is for the purpose of linkage with all the loT devices and has a feature of realizing a constant connection function and a directory function.

The constant connection function is a function of constantly connecting usable loT services (IoT applications 1700) through the first device 1410, the second device 1510, the third device 1610, and the loT hub 1200.

Here, there are two types of loT device connected to the loT hub including one type for which constant connection is not essential and the other type for which constant connection is essential. In the former, a device of a sensor system regularly or irregularly transmitting information is included. In the latter, a device such as a controllable device for which a remote-control operation needs to be performed is included. Thus, the constant connection function described above according to the present disclosure may realize constant connection for an loT device of the latter for which constant connection is essential and realize connection only on request for an loT device of the former for which constant connection is not essential.

The constant connection function can be realized by using a communication protocol such as loT-dedicated Message Queue Telemetry Transport (MQTT) for the loT hub 1200 according to the present disclosure, the first device 1410, the second device 1510, the third device 1610, and the loT service 1700.

In addition, a directory function links the first device 1410, the second device 1510, the third device 1610, and the loT service to each other.

In other words, the directory function realizes a function for specifying a mono or a service from a certain mono (device) to a certain service, from a certain service to a certain mono, or from a certain mono to another mono and instructing the mono or the service.

The directory function can specify a device of a linkage destination designated by a device of a linkage source on the basis of identification information of devices, identification information of drivers, and identification information of connection interfaces.

The identification information of a device is a UUID of an loT device. The identification information of a driver is an ID of the driver. The identification information of a connection interface is information used for specifying an R interface that is an interface between a driver and the loT hub 1200.

In addition, the directory function can specify a device of a linkage destination on the basis of the identification information of the loT hub 1200.

In addition, the identification information of devices, the identification information of drivers, and identification information of connection interfaces are stored in a predetermined storage device, and the storage device may store identification information of connection source devices and/or functions of devices to which connection is not permitted as a white list. In such a list, a menu vote of a support method to be described below may be used.

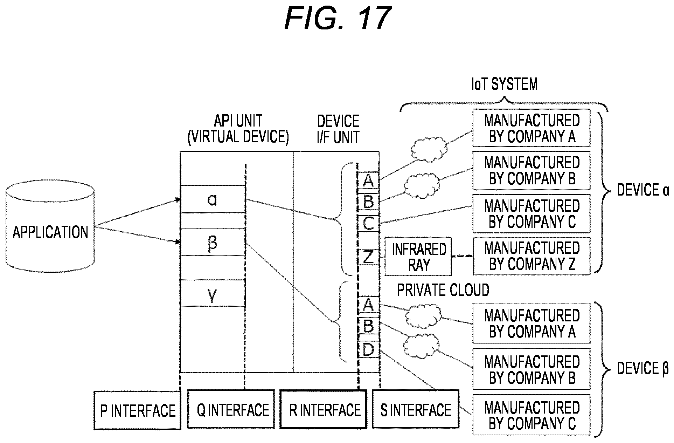

FIG. 17 is a conceptual diagram illustrating a mutual connection infrastructure in an loT connection system according to the present disclosure. As illustrated in FIG. 17, although a connection face between loT applications represented as applications and an loT hub are P interfaces, by connecting them using WebAPI (α, β, γ), the connection faces may have standardized Q interfaces.

Similarly, although connection faces between loT devices and an loT hub are S interfaces, by connecting them using drivers (A to Z), the connection faces may have standardized R interfaces.

Conventionally, in order for an IoT application to transmit/receive information to/from an embedded application of an loT device, an loT device that is a destination needs to be designated on the loT application side, and identification information that can be designated from an loT application needs to be included also on the loT device side.

On the other hand, in the loT connection system according to the present disclosure, the designation of a destination described above is performed using a directory function realized by the loT hub.

For example, addition or replacement of an loT device can be handled only by adding a driver to the loT hub and adding or replacing a connection destination, and the loT application does not need to be corrected.

In other words, a destination part designating an loT device that is necessary in an loT application and an identification information part that can be designated from an loT application that is necessary in an loT device can be aggregated in a certain loT hub.

For this reason, addition or replacement of an loT device does not increase the number of processes of an application developer, which leads to reduction of the cost.

In this way, according to the loT connection system of the present disclosure, even in a case in which manufacturers and models of loT devices are different from each other, by connecting them to an IoT hub, protocol-free mutual connection can be realized.

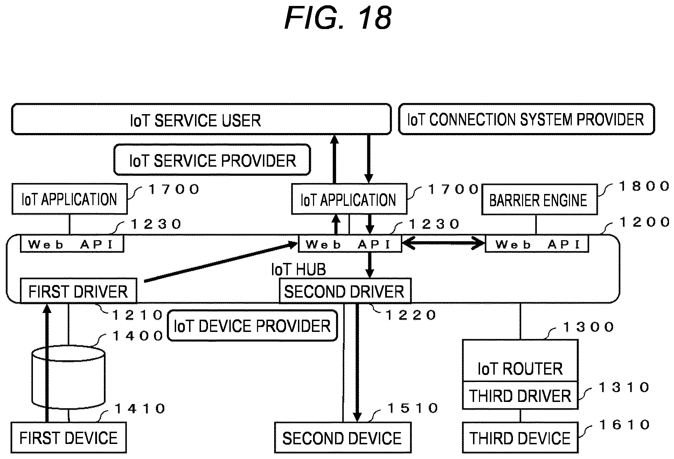

In addition, as illustrated in FIG. 18, the loT hub 1200 according to the present invention can be connected to a barrier engine 1800. This barrier engine 1800 is used for checking details of an instruction of the loT hub 1200 for a device and preventing execution of an inappropriate action.

For example, in a case in which an loT service "when outer air is fresh, the air conditioner is stopped, and the window is opened" is provided, when it is struck by a Guerrilla storm immediately after that, there is concern that an indoor place becomes wet, and the barrier engine 1800 described above is for preventing such a threat originated from an loT in advance.

In addition, at least one of the first driver 1210, the second driver 1220, and the third driver 1310 according to the present invention can realize a virtual device function.

This virtual device function virtually reproduces the first device 1410, the second device 1510, or the third device 1610.

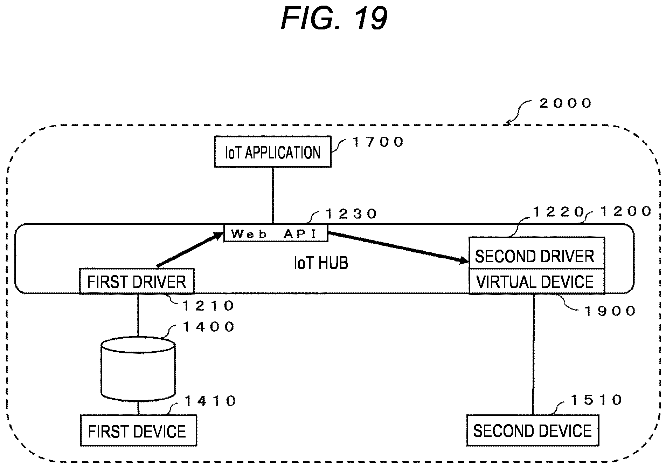

FIG. 19 illustrates an example in which the second driver 1220 according to the present invention realizes a virtual device function. As illustrated in FIG. 19, by disposing a virtual device 1900 that can reproduce transmission/reception of a command of the second device 1510 in the second driver 1220, even if the second device 1510 is not connected, an IoT application using the second device 1510 can be developed. In addition, in a case in which there is an operational failure, without having a visit to a field, failure separation relating to which one of the second device 1510 and the loT hub 1200 has failed can be easily performed.

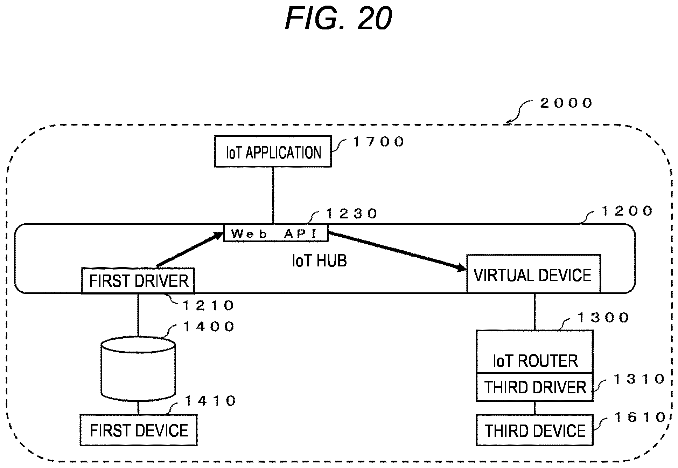

In addition, in the present invention, as illustrated in FIG. 20, a virtual device function may be implemented in not a driver but the loT hub 1200. This is an effective means for isolation of a failure of the third device 1610 connected to the loT router 1300 that is locally present.

As described above, the loT hub according to the present disclosure is a protocol-free infrastructure that enables mutual connection of clouds using an interface called a driver even in a case in which the clouds use different communication protocols. In addition, by using the directory function, connection can be made by complicatedly combining a plurality of applications and loT devices.

In the loT connection system 2000 according to the present invention, information acquired from the first device 1410, the second device 1510, or the third device 1610 may be configured not to be stored in the loT hub 1200.

The loT connection system 2000 according to the present invention is assumed to be operated by a telecommunication company. Since an obligation of obeying confidentiality of communication is imposed on a telecommunication company, information acquired from various devices is not stored for the purpose of other uses.

Such information is useful information, and thus, in order to exclusively acquire such information, generally, enclosure of loT devices is performed in a private cloud of each company.

On the other hand, the loT connection system 2000 according to the present invention is operated by a telecommunication company, and thus, by mutually connecting loT devices and loT applications in a neutral position, an loT business can be promoted.

In addition, the continuity of an loT mutual-connection service has an influence on the continuity of an loT service of a using company, and thus it is preferable that using companies share the mutual connection infrastructure together.

In addition, in the loT connection system 2000 according to the present invention, only devices and loT applications permitted using an API key and an authentication scheme can be configured to be able to be connected to the loT hub 1200. In other words, the loT connection system 2000 according to the present invention can build an loT communication-dedicated closed network on the Internet. In addition, since a communication path is also encrypted, and an loT router is managed using a technique of Mobile Device Management (MDM), a new attack method and weakness of an OS and an application can be handled.

Furthermore, in order to connect a device that is not formed as loT to the loT hub 1200, by using a BaaS (Backend as a service) and an SDK, development can be performed in a short period.

According to the configuration described above, technical improvement for solving or alleviating at least some of the problems of the conventional technology described above can be provided. In addition, according to the configuration described above, creation of a new service combining functions of a plurality of devices can be efficiently performed.

As illustrated in FIG. 8, the server apparatus 300 can be configured to include a transmission unit 360.

The transmission unit 360 transmits information about a release key that can release a specific electronic lock only to the first information processing apparatus 100 corresponding to first identification information received from the second information processing apparatus 200.

In other words, only persons who have an application installed in their smartphones can be permitted to enter/exit a conference room or the like.

As above, in the embodiment described above, although description has been made by assuming that the second information processing apparatus 200 is a device having a function of receiving a signal and is installed in a conference room or the like inside a company, and the first information processing apparatus 100 is a device such as a smartphone or the like having a function of transmitting signals and is held by a user, such transmission and reception functions thereof may be opposite thereto. In other words, the first information processing apparatus 100 may have a function of receiving a signal, and the second information processing apparatus 200 may have a function of transmitting signals.

In addition, both the first information processing apparatus 100 and the second information processing apparatus 200 may have both functions including the transmission function and the reception function. In such a case, the function implemented by each apparatus can be remotely automatically switched on the basis of an instruction from the server apparatus 300.

In addition, the apparatus implementing the transmission function (the first information processing apparatus 100 in the embodiment described above) is not limited to an information processing apparatus such as the smartphone or the like described above, and a transmitter of a tag type (a so-called "signal tag") can be used.

In accordance with this, behavior histories of a child and an old person who do not/cannot hold a smartphone and a medical/nursing staff who cannot carry a smartphone can be acquired easily and reliably, and persons present at the same place as that of the persons described above can be specified easily and reliably.

Hereinafter, an embodiment of a service realized by an information processing system, an information processing method, and a computer program according to the present disclosure will be described.

### <Overview>

According to a service implemented by an information processing system, an information processing method, and a computer program of the present disclosure, an loT sensor is installed in a conference room and the like, and an employee who has entered the conference room at a predetermined time can be specified using a smartphone held by him or her.

When an infected person infected with the novel coronavirus or the like was found among employees, a close contact person or a contact infection possible person can be specified using the information. A means for preventing spread of infection such as causing not all the employees who entered/exited a building but only close contact persons or contact infection possible persons to stay at home or the like can be easily taken.

Besides, by embedding various sensors such as an indoor environment sensor and the like as loT sensors, a countermeasure for performing continuous monitoring of an indoor environment and preventing indoor spread of infection through appropriate ventilation and the like as possibly can be also employed.

### <Provision method>

In providing this service, an loT sensor and a gateway (an loT router) for allowing the sensor to communicate with the Internet are provided. In addition, also among loT sensors, a function for specifying an employee entering a room by broadcasting a BLE signal is built in an loT router.

Furthermore, in order to specify that an employee has entered a room by receiving a BLE signal, an application according to the present disclosure needs to be installed and registered in a company or personal smartphone in advance.

Room entrance information and indoor environment information collected using this structure are accumulated in a "Corona Tracer" service through "loT Exchange" to be described below in detail and can be freely drawn by a staff of each company.

In addition, for acquisition of information and an action based on the information, an automated tool such as robotic process automation (RPA) or the like may be used.

### <Constituent element of service and function thereof>

"loT Exchange" transmits and receives commands for controlling sensors and the like and information transmitted from sensors.

"IoT router" is responsible for connection with an environment sensor and not only transmits environment data to "loT Exchange", but a built-in BLE chip achieves roles of BLE transmitter/receiver and can receive a BLE signal transmitted from a smartphone and transmit a BLE signal.

The "environment sensor" measures environment data (a room temperature, humidity, noise, an amount of CO2, an amount of volatile organic solvent, and the like) of a conference room and the like and transmits the environment data to the "Corona Tracer" service by way of the loT router.

"Corona Tracer App" corresponds to iOS and Android, is installed in a smartphone (provided by the company or personally owned) held by an employee and transmits a BLE signal. In addition, the "Corona Tracer App" transmits checking information representing whether the BLE signal is constantly transmitted to a "Corona Tracer" service. Furthermore, the "Corona Tracer App" may receive a BLE signal and transmit the information (a time stamp, a company ID, an employee ID, a signal ID, and a signal intensity) to the "Corona Tracer" service.

The "Corona Tracer" service collects information from the environment sensor, the loT router, and the "Corona Tracer App" and stores the information. In addition, this service can remotely perform setting of the loT router. Furthermore, in a case in which the loT router is used as a BLE transmitter, an early output interval and a transmission intensity of BLE can be changed. This service can further generate a report for entrance/exit information of an employee for a conference room and store the information in a csv file.

"RPA" or another system desiring linkage accesses the "Corona Tracer" service, acquires conference room entrance/exit information of an employee, and generates a report or transmits an alert on the basis of the information.

### <Preparation of service provision>

In provision of this service, installation of devices in a conference room, the employee's installation of an application, and various registrations in the RPA or another system desiring linkage are necessary. The devices installed in the conference room are an loT router, an environment sensor, and an AC adaptor. In a case in which a power supply is not present in an indoor place, a mobile battery is necessary as well. Although the loT router is an Android smartphone, kitting such as activation, installation of an application, and the like are performed by this service provider. Thus, an installation company staff may only place a complete set of this device in the conference room.

The application installed in a smartphone of an employee is downloaded by each employee from Google Play Store or Apple App Store and is installed. In addition, after installation, a company ID and an employee ID need to be registered.

The application described in the prior art literature is premised that the application is installed in smartphones of both parties, according to the invention of the present disclosure, the other party may operate also in accordance with a tag or the like. In accordance with this, also a child or an old person not having a smartphone and a medical/nursing staff who is unable to carry a smartphone can be handled.

In addition, although the application described in the prior art literature is of only a type that is stored in smartphones, according to the invention of the present disclosure, a method in which the application is stored in both a smartphone and a cloud can be selected. In addition, relative distance information of both parties and information that can be used for specifying the person are separated as completely separate systems, and there is such a restriction that only an access by way of a linkage system can be performed. In accordance with this, although information of the terminal side and information of the cloud side were able to be accessed, the information cannot be obtained.

In addition, although the application described in the prior art literature is an application for a smartphone, has no concept of a parent device and a child device, and is premised that any terminal is equal and does not acquire position information, according to the invention of the present disclosure, the reception side can be installed at a specific place and thus a group infection place can be easily specified. In accordance with this, a place and a date and time at which a group infection has occurred can be easily specified.

More specifically, examples of the place include a conference room, a restroom, a store, a club, a classroom, a karaoke room, and the like. When the place and the date and time can be specified, even in a case in which a person who was present at the place is a person who does not have a smartphone, an application, a tag, or the like, there are cases in which an infection risk is able to be notified. In addition, according to the invention of the present disclosure, a scheme in which, even in a case in which there are several child devices, a parent device receives data is employed, and thus data accumulating/collecting places can be limited and uniformized. The parent device is managed with being introduced and installed in a conference room or the like by a company, and thus a place (including position information) of the parent device can be specified, and it is preferable that the parent device be able to be remotely operated and maintenance by the company.

In addition, although the application described in the prior art literature is necessarily required and is difficult to be linked with other similar applications and services, according to the invention of the present disclosure, systems that are individually introduced by a specific company, a specific organization, a specific self-governing body, and the like can be connected to each other in a simple manner, and only information based on a contract with the other party with which the contract has been formed can be shared. In accordance with this, it can be assured that information that is not based on a personal agreement or a contract is not shared.

In addition, although the application described in the prior art literature is a general infection information sharing system of a scheme in which, basically, personal information is not specified at all, according to the invention of the present disclosure, information of a place installed by a company can be added, and thus it can be specified which place becomes an infection place. As a result, the company reduces a risk of stopping the business, and the safety of employees can be secured. The company described here represents all the companies such as a company (a conference room, a restroom), a school (a classroom, a restroom), a store (foods, Karaoke, a club, and the like) and the like that have been unable to individually set up a countermeasure until now.

In addition, by being linked with a Global Positioning System (GPS) function of the first information processing apparatus, the invention of the present disclosure may build a structure in which, in a case in which an infected person has occurred in the vicinity of a person who was instructed to stay at home after returning to the country, in order to approve that he or she had continuously stood by at home, his or her position information can be presented.

Finally, embodiments of an information processing method and a computer program according to the present disclosure will be described.

As illustrated in FIG. 1, the information processing method according to the present disclosure is an information processing method executed in the information processing system 1000 including the second information processing apparatus 200 having a function of receiving a signal transmitted from the first information processing apparatus 100 and the server apparatus 300 that can be connected to at least the second information processing apparatus 200 through the Internet.

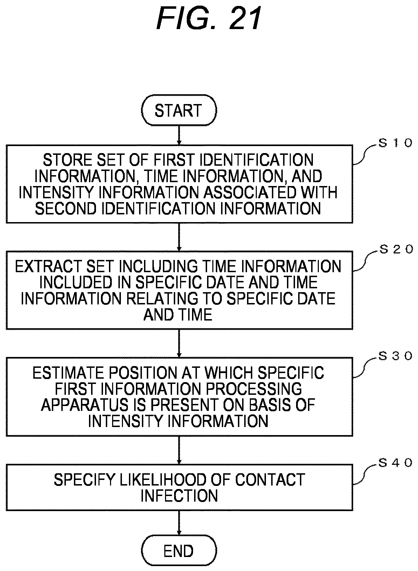

As illustrated in FIG. 21, the information processing method according to the present disclosure causes the server apparatus 300 to execute a storage step S10, an extraction step S20, an estimation step S30, and a specifying step S40.

In the storage step S10, a set of at least first identification information of one or more first information processing apparatuses transmitting a signal received by the second information processing apparatus, time information at which the signal was received, and intensity information of the signal is stored in association with second identification information of the second information processing apparatus. Such a storage step S10 can be executed by the storage unit 310 described above. Details of the storage unit 310 are as described above.

In the extraction step S20, in a case in which the server apparatus receives designation of specific first identification information of a specific first information processing apparatus among one or more first information processing apparatuses and a specific date and time, a set including time information included in specific date and time information relating to the specific date and time is extracted among sets in which the specific first identification information is included. In such an extraction step S20 can be executed by the extraction unit 320 described above. Details of the extraction unit 320 are as described above.

In the estimation step S30, a position at which the specific first information processing apparatus was present is estimated on the basis of intensity information included in the set extracted in the extraction step. Such an estimation step S30 can be executed by the estimation unit 330 described above. Details of the estimation unit 330 are as described above.

In the specifying step S40, another user having another first information processing apparatus estimated to have been present at approximately the same position as the position estimated in the estimation step after the date and time at which the specific first information processing apparatus was present is specified as a contact infection possible person. Such a specifying step S40 can be executed by the specifying unit 340 described above. Details of the specifying unit 340 are as described above.

According to the configuration described above, technical improvement for solving or alleviating at least some of the problems of the conventional technology described above can be provided.

The computer program according to the present disclosure is a computer program executed in an information processing system including a second information processing apparatus having a function of receiving a signal transmitted from a first information processing apparatus and a server apparatus that can be connected to at least the second information processing apparatus through the Internet.

The computer program according to the present disclosure causes the server apparatus 300 to implement a storage function, an extraction function, an estimation function, and a specifying function.

The storage function stores a set of at least first identification information of one or more first information processing apparatuses transmitting a signal received by the second information processing apparatus, time information at which the signal has been received, and intensity information of the signal in association with second identification information of the second information processing apparatus.

The extraction function, in a case in which the server apparatus receives designation of specific first identification information of a specific first information processing apparatus among one or more first information processing apparatuses and a specific date and time, extracts a set including time information included in specific date and time information relating to the specific date and time among sets in which the specific first identification information is included.

The estimation function estimates a position at which the specific first information processing apparatus was present on the basis of intensity information included in the set extracted in the extraction function.

The specifying function specifies another user, who has another first information processing apparatus, estimated to have been present at approximately the same position as the position estimated using the estimation function after the date and time at which the specific first information processing apparatus was present as a contact infection possible person.

The storage function, the extraction function, the extraction function, and the specifying function can be respectively executed by a storage circuit, an extraction circuit, an estimation circuit, and a specifying circuit. Such circuits can be configured to be included in the storage unit 310, the extraction unit 320, the estimation unit 330, and the specifying unit 340 described above. Details thereof are as described above.

According to the configuration described above, technical improvement for solving or alleviating at least some of the problems of the conventional technology described above can be provided.

Finally, another application example of the information processing system according to the present disclosure will be described.

The novel coronavirus is reported to be infected mainly through a splash contact with an infected person and the like, and a health services administration requests cutting out an infection path for inhibiting spread of infection and quickly specifying a close contact person in the case of infection through inevitable contact.

For this reason, the government and infectious disease research organizations open places (facilities such as a store, an office, and the like) at which there were uses and the like of infected persons, and a company providing a service enabling checking of such places on a map using position information of a GPS or the like and, as disclosed in Non-Patent Literature 1, a service for tracking an infection path and notifying a close contact person of the infection path have appeared.

In the service disclosed in Non-Patent Literature 1, when smartphones in which a corresponding application has been installed become close to each other, IDs assigned to terminals are exchanged using "Bluetooth" of near field communication and are recorded. When an infected person is an application user, after an approval is obtained from the person by a staff of a health department, smartphone users who had been near the infected person for a predetermined time or more are specified and contacted on the basis of recordings in the application, and a necessary countermeasure is taken.

By utilizing such an application, close contact persons not recognized by an infected person can be tracked, and there is an effect of early detection of a cluster (a group of infected persons) and early isolation of persons having likelihoods of infection. However, this application is premised on the use of smartphones, and, when many persons do not use the application, the effect is not raised.

At present, as a countermeasure for spread of infection of the novel coronavirus, an emergency situation declaration was issued, and many companies are requesting refrainment from going out. However, there are tasks that cannot be handled at home and business types that are necessary for maintaining a social function, and persons participating in such tasks are forced to be in a situation in which continuous commuting needs to be continued.

Meanwhile, currently, infected persons are detected in companies, stores, hospitals, and the like performing tasks that are necessary for securement of stability of daily lives of people/national economy, and a situation in which not only specific tasks but the business needs to be temporarily stopped occurs.

As a countermeasure for a business continuation plan (BCP), it has already been essential to quickly specify close contact persons and classify them in all the companies.

Thus, for the purpose of a countermeasure for a BCP plan of a company, the present inventor has developed a "close contact person specifying service" by utilizing the loT router 1300 according to the present disclosure installed in a conference room or the like as the second information processing apparatus 200.

First, the loT router (parent device: the second information processing apparatus 200) according to the present disclosure is installed at a place such as a conference room of a company in which "three closings" may easily occur. When an employee having a smartphone (child device: the first information processing apparatus 100) in which a beacon application has been installed becomes close thereto, a child device UUID and a distance of the employee are detected, and the child device UUID and the distance are recorded together with a time.

For this reason, only by designating an infected employee's name, a manager of a general affairs department or the like can simply specify employee's names having a likelihood of close contact.

This "close contact person specifying service" has a structure in which a necessary amount of necessary data is acquired at a necessary time from different systems including a "beacon data collecting system" and a "close contact person specifying system" through the "loT hub" and the "loT router" and has a structure for which the manager cannot continuously monitor a someone's behavior.

In other words, a system including the first information processing apparatus 100 and the second information processing apparatus 200 according to the present disclosure is the "beacon data collecting system", a system including the server apparatus 300 is the "close contact person specifying system", and the loT hub 1200 described above functions as a hub connecting these.

For this reason, both high-level privacy protection and securement of security can be achieved. In other words, two systems including the "beacon data collecting system" in which data to be collected is limited to data not corresponding to personal information and the "close contact person specifying system" handling personal information such as names and contact points of employees are built, and information is blocked by dividing these two systems.

Then, by loosely coupling such systems using the loT hub only when it is necessary, close contact persons can be specified while constant monitoring of the behaviors of employees is prevented.

In such a system configuration, by connecting systems of various functions to the IoT hub 1200, the function can be expanded.

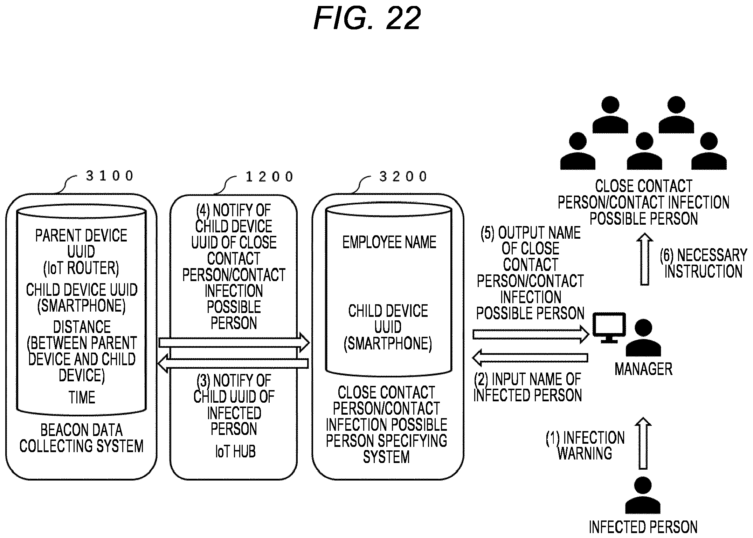

Subsequently, a flow of specifying close contact persons from an occurrence of an infected person will be described with reference to FIG. 22.

More specifically, in the beacon data collecting system 3100 illustrated in FIG. 22, data (a UUID, a time, and a distance between the loT router that is a parent device and the smartphone that is a child device) of persons present in a conference room is automatically collected in advance using a beacon installed in the conference room and a smartphone application. In this beacon data collecting system 3100, personal information is not included at all.

Then, when a manager receives a report of infection from an employee who becomes an infected person, the manager inputs a name of the employee from a management screen. Then, the close contact person specifying system 3200 transmits a UUID of the infected person to the beacon data collecting system 3100. The beacon data collecting system 3100 specifies a UUID having a likelihood of close contact on the basis of this UUID and transmits the specified UUID to the close contact person specifying system 3200. Then, this system 3200 outputs the UUID and the name in association with each other as a close contact person candidate list. A staff of the general affairs department who is the manager contacts close contact person candidates with priority levels assigned thereto from information of the list and gives an instruction for waiting at home and receiving a test and an instruction for continuous reporting, and the like.

In addition, in the "Corona Tracer", BLE is used as a beacon. Although a close contact can be detected between smartphones using a beacon, by considering that there is an employee not having a smartphone, and some smartphones cannot perform bidirectional communication of BLE, the loT router is used as a parent device, and an employee who cannot perform BLE bidirectional communication is allowed to have a beacon tag, whereby all the employees can use the system. Data collected utilizing BLE between smartphones is utilized for an auxiliary use such as improvement of accuracy of the distance and the like.

In addition, the manager of this system notifies an employee of an ID and a password through a mail. In a case in which an employee who has received the notification inputs an ID and a password that have been transmitted when installing the application, a UUID is automatically generated in the smartphone and is registered in the close contact person specifying system together with the name. In the beacon data collecting system, only this UUID is transmitted and is used for recording, and thus a person cannot be specified from the UUID.

In addition, by using the loT hub, data of another company can be shared in a limited manner as information on the basis of a contract. By utilizing this function, it can be taught that there is a risk of infection also for an employee of another company who sit together.

In addition, when an introduced company agrees, by laying down personal information, the information can be provided altogether for the government, a self-governing body, or an insurance organization. In accordance with this, infected person group (cluster) information can be quickly provided.

Finally, an example in which a medium having a two-dimensional code printed thereon is used in place of or in addition to the first information processing apparatus 100 will be additionally described.

In the embodiment described above, although a person having the first information processing apparatus 100 in which a dedicated application is installed is assumed to be a target person in description, there are many cases in which a person having another information processing apparatus (an information processing terminal in which a dedicated application has not been installed), a person having no information processing terminal, and the like visit a company.

For example, a worker performing a construction work and the like and a visitor such as a person temporarily visiting for a conference or the like can be more easily managed by printing a two-dimensional code such as a QR code (registered trademark) in an entrance ticket and managing information included in this two-dimensional code than in a case in which management is performed using the smartphone in which the dedicated application has been installed and the signal tag described above.

In this QR code, information of a name and a contact point (a mail address and/or a telephone number and the like) of a visitor is recorded. Such information may be input by a visitor through a predetermined input terminal at the time of visiting or may be registered in advance by a visitor or a person of a visit reception side.

Then, the visitor causes the second information processing apparatus 200 that is a parent device installed in the conference room to read a two-dimensional code printed on his or her entrance ticket when entering the conference room. In accordance with this, information of the visitor and information of the read time and the like are recorded in the server apparatus 300.

The two-dimensional code is not limited to being read by the parent device, and a form in which the two-dimensional code is read by the first information processing apparatus 100 that is a child device and is transmitted to the parent device may be employed.

Then, in a case in which there is need to contact the visitor in combination with the embodiment described above, it is preferable that the contact be made by transmitting an email or a short message service (SMS) to a mail address or a telephone number recorded as contact points.

In addition, the two-dimensional code described above can be utilized as an online name card. In other words, in place of exchange of conventional name cards for exchanging actual paper media, by recording details of the name card in a two-dimensional code and displaying the details on a screen of a smartphone or the like, exchange of name cards can be remotely performed with a contact being avoided.

In addition, by displaying online name cards on a screen shared by participants in a conference, exchange of name cards with participants can be performed at the same time as well.

Although several embodiments of the present invention have been described, such embodiments are presented as examples and do not intend to limit the scope of the invention. These novel embodiments can be performed in other various forms, and various omissions, substitutions, and changes can be performed in a range not departing from the gist of the invention. These embodiments and modifications thereof are included in the scope and the gist of the invention and are included in the invention described in the claims and an equivalent range thereof.

In addition, the techniques described in the embodiments may be stored, for example, on a recording medium such as a magnetic disk (a floppy (registered trademark) disk, a hard disk, or the like), an optical disc (a CD-ROM, a DVD, an MO, or the like), a semiconductor memory (a ROM, a RAM, a flash memory, or the like) or the like and can be transmitted and distributed using a communication medium as a program that can be executed by a computer. In addition, a program stored on the medium side also includes a setting program configuring a software means (including not only an execution program but also a table and a data structure) to be executed by a computer inside the computer. A computer realizing the present apparatus reads a program recorded on a recording medium, builds a software means using a setting program in accordance with a case, and has the operation controlled by this software means, thereby executing the process described above. In addition, a recording medium described in this specification is not limited to a recording medium for distribution but includes storage medium such as a magnetic disk, a semiconductor memory, or the like installed inside the computer or in a device connected through a network. The storage unit, for example, may function as a main storage device, an auxiliary storage device, or a cache memory device.

### [REFERENCE SIGNS LIST]

1000 Information processing system
100 First information processing apparatus
110 Transmission unit
200 Second information processing apparatus
210 Reception unit
220 Transmission unit
230 Acquisition unit
240 Environment information transmitting unit
300 Server apparatus
310 Storage unit
320 Extraction unit
330 Estimation unit
340 Specifying unit
350 Notification unit
360 Transmission unit

## Claims

1. An information processing system comprising:
a second information processing apparatus having a function of receiving a signal transmitted from a first information processing apparatus; and
a server apparatus that is connectable to at least the second information processing apparatus through the Internet,
wherein the server apparatus includes:
a storage unit storing a set of at least first identification information of one or more first information processing apparatuses transmitting a signal received by the second information processing apparatus, time information at which the signal has been received, and intensity information of the signal in association with second identification information of the second information processing apparatus;
an extraction unit extracting a set including time information included in specific date and time information relating to a specific date and time among sets in which specific first identification information is included in a case in which the server apparatus receives designation of the specific first identification information of the specific first information processing apparatus among one or more first information processing apparatuses and the specific date and time;
an estimation unit estimating a position at which the specific first information processing apparatus was present on the basis of intensity information included in the set extracted by the extraction unit; and
a specifying unit specifying another user having another first information processing apparatus estimated to have been present after a date and time at which the specific first information processing apparatus was present at approximately the same position as the position estimated by the estimation unit as a contact infection possible person.

2. The information processing system according to claim 1,
wherein the specifying unit specifies another first information processing apparatus estimated to have been present at approximately the same position as the position estimated by the estimation unit between the date and time at which the first information processing apparatus was present and a predetermined end date and time, and
wherein the server apparatus sets the predetermined end date and time on the basis of third identification information received from a third information processing terminal and a reception time of the third identification information.

3. The information processing system according to claim 1 or 2, wherein the specifying unit specifies another user having another first information processing apparatus that is estimated to have been present at the date and time at which the specific first information processing apparatus was present within a predetermined range from the position estimated by the estimation unit as a close contact person.

4. The information processing system according to claim 1, 2, or 3,
wherein the server apparatus further includes a notification unit notifying a user having another first information processing apparatus having another first identification information specified by the specifying unit of specific information, and
wherein the notification unit notifies a user having another first information processing apparatus having another first identification information not associated with denial information representing that a countermeasure for preventing contact infection has been performed among other pieces of first identification information specified by the specifying unit of specific information.

5. The information processing system according to claim 2, wherein, by reading a two-dimensional barcode displayed in a medium installed for each predetermined area, the third information processing terminal transmits the third identification information to the server apparatus.

6. The information processing system according to any one of claims 1 to 5, wherein the specific date and time information includes a date and time that is a first period before the specific date and time.

7. The information processing system according to claim 6,
wherein the specific date and time is a date and time at which a specific user using the specific first information processing apparatus has been diagnosed to have a predetermined infectious disease, and
wherein the first period is an incubation period of the infectious disease.

8. The information processing system according to claim 1, the specific date and time information includes a date and time that is a second period after the specific date and time.

9. The information processing system according to any one of claims 1 to 8,
wherein a plurality of second information processing apparatuses are installed in a predetermined area, and
wherein the estimation unit estimates a position at which the specific first information processing apparatus was present on the basis of intensity information included in a set extracted from the plurality of second information processing apparatuses.

10. An information processing method executed in an information processing system including a second information processing apparatus having a function of receiving a signal transmitted from a first information processing apparatus and a server apparatus that is connectable to at least the second information processing apparatus through the Internet, the information processing method causing the server apparatus to execute:
a storage step of storing a set of at least first identification information of one or more first information processing apparatuses transmitting a signal received by the second information processing apparatus, time information at which the signal was received, and intensity information of the signal in association with second identification information of the second information processing apparatus;
an extraction step of extracting a set including time information included in specific date and time information relating to a specific date and time among sets in which specific first identification information is included in a case in which the server apparatus receives designation of the specific first identification information of the specific first information processing apparatus among one or more first information processing apparatuses and the specific date and time;
an estimation step of estimating a position at which the specific first information processing apparatus was present on the basis of intensity information included in the set extracted in the extraction step; and
a specifying step of specifying another user having another first information processing apparatus estimated to have been present at approximately the same position as the position estimated in the estimation step after the date and time at which the specific first information processing apparatus was present as a contact infection possible person.

11. A computer program executed in an information processing system including a second information processing apparatus having a function of receiving a signal transmitted from a first information processing apparatus and a server apparatus that is connectable to at least the second information processing apparatus through the Internet, the computer program causing the server apparatus to implement:
a storage function of storing a set of at least first identification information of one or more first information processing apparatuses transmitting a signal received by the second information processing apparatus, time information at which the signal was received, and intensity information of the signal in association with second identification information of the second information processing apparatus;
an extraction function of extracting a set including time information included in specific date and time information relating to a specific date and time among sets in which specific first identification information is included in a case in which the server apparatus receives designation of the specific first identification information of the specific first information processing apparatus among one or more first information processing apparatuses and the specific date and time;
an estimation function of estimating a position at which the specific first information processing apparatus was present on the basis of intensity information included in the set extracted in the extraction function; and
a specifying function of specifying another user having another first information processing apparatus estimated to have been present at approximately the same position as the position estimated in the estimation function after the date and time at which the specific first information processing apparatus was present as a contact infection possible person.
